Europäisches Patentamt

European Patent Office

Office européen des brevets

(11) Publication number: **0 351 974**
**A1**

## (12) EUROPEAN PATENT APPLICATION

(21) Application number: **89306686.0**

(22) Date of filing: **30.06.89**

(51) Int. Cl.4: **A61K 37/66 , //(A61K37/66, 31:365)**

(30) Priority: **05.07.88 US 215013**

(43) Date of publication of application:
**24.01.90 Bulletin 90/04**

(84) Designated Contracting States:
**ES GR**

(71) Applicant: **SCHERING CORPORATION**
**2000 Galloping Hill Road**
**Kenilworth New Jersey 07033(US)**

(72) Inventor: **Peets, Edwin A.**
**160 West 96th Street**
**New York New York 10025(US)**
Inventor: **Taylor, Eugene L.**
**15 Wesley Avenue**
**Benardsville New Jersey 07924(US)**
Inventor: **Smiles, Kenneth A.**
**734 Sutro Avenue**
**Novato California 94947(US)**
Inventor: **Tanner, Daniel J.**
**1401 55th Street**
**Brooklyn New York 11219(US)**

(74) Representative: **Ritter, Stephen David et al**
**Mathys & Squire 10 Fleet Street**
**London EC4Y 1AY(GB)**

(54) **Treatment of genital warts with a combination of podophyllin and recombinant DNA human alpha interferon.**

(57) Condylomata acuminata infections (genital warts) are treated in infected patients by concomitantly administering recombinant-DNA human alpha-interferon and podophyllin once a week for three weeks.

The interferon exemplified is recombinant-DNA human interferon alfa-2b in which $1.5 \times 10^6$ International Units are administered by injection to each lesion.

The podophyllin exemplified is a 25% solution in tincture of benzoin administered topically to each lesion.

EP 0 351 974 A1

# TREATMENT OF GENITAL WARTS WITH A COMBINATION OF PODOPHYLLIN AND RECOMBINANT DNA HUMAN ALPHA INTERFERON

## BACKGROUND

### 1. FIELD OF INVENTION

This invention relates to a method of treating condyloma acuminatum (genital warts) with a combination of podophyllin, or an active constituent thereof, and recombinant-DNA human alpha-interferon (hIFN-α). The combination treatment displays greater effects than the treatment of condyloma acuminatum with podophyllin alone.

### 2. PRIOR ART

Condyloma acuminatum is a venereal disease caused by human papilloma viruses (HPV), including e.g. Types 6 and 11. The disease is usually referred to as genital warts. The incidence of the disease is increasing and there is evidence linking HPV infections to genitourinary neoplasms.

Podophyllum resin, more commonly referred to as podophyllin, has been considered the treatment of choice for condyloma acuminatum by many physicians, primarily because of its ease of application, i.e. topically, and low cost per treatment. A principal active constituent in podophyllin is podophyllotoxin.

Although podophyllin is relatively inexpensive and easy to use, it causes local tissue destruction, its efficacy is limited and, if used for extended periods of time, it has toxic side effects. Recommended podophyllin therapy for condyloma acuminatum includes topical application of 25% podophyllin in tincture of benzoin once a week for no longer than three weeks. This is described by Miller, R.A., International Journal of Dermatology, 24, 491-498 (1985). Results are often unsatisfactory since the lesions of the disease either do not completely regress or recur within four months. As discussed by Ferenczy et al., The New England Journal of Medicine, 313: 784-788 (1985), who used laser therapy, the HPV is present in clinically and histologically normal squamous epithelium beyond areas treated destructively. Such presence of the virus in the epithelium was shown to correlate strongly with the risk of recurrence.

Human alpha-interferon is a naturally occurring mixture of at least eleven compounds including those designated alpha-1 interferon and alpha-2 interferon. Alpha-interferon exhibiting biological properties similar to those of naturally occurring human leukocyte interferon can be made by recombinant methods.

A number of alpha interferon species or components are known and are usually designated by a numeral and letter after the Greek letter alpha. Human alpha-1 interferon is one species appropriate for use in this invention, as are the species designated human alpha-2 interferons. Under USAN (U.S. Approved Names), recombinant-DNA human alpha-2 interferons are designated Interferon Alfa-2a, which can be made as disclosed by Rubenstein, Biochem. Biophys. Acta 695, 5-16 (1982), and Interferon Alfa-2b. Interferon Alfa-2b is the preferred species for use in this invention and is a recombinant-DNA human alpha-interferon. Also suitable is recombinant-DNA human interferon alfa-2a.

Human interferon alfa-2b can be produced in bacteria and other microorganisms using recombinant-DNA techniques including those disclosed by Nagata et al. Nature, 284, 316-320 (1980), European Patent 32,134, and US Patent No. 4,289,690. Various alpha interferon species are disclosed in US patent 4,503,035. The preferred human interferon alfa-2b used in this invention is also denoted herein as "hIFNα-2b". Intralesionally administered interferon has been shown to clear about one third of cases of condylomata acuminata when given three times weekly for three weeks as indicated by Eron et al., New England Journal of Medicine 315, 1059-1064, (1986) and Smiles et al., The Biology of the Interferon System, 1986, Cantell et al., editors, Dordrecht, Martinus Mijhoff Publishers, 493-501 (1987). Friedman-Kien et al., JAMA, 259, 533-538 (1988) reported that intralesional interferon has been shown to clear about two-thirds of cases when given twice weekly for up to eleven weeks.

## SUMMARY OF THE INVENTION

This invention relates to a method of treating condylomata acuminata with a combination of topical podophyllin and injectable interferon. This invention provides, in its preferred aspects, compositions and methods for treating condylomata acuminata utilizing (1) a topical pharmaceutically acceptable composition comprising a solution of podophyllin in tincture of benzoin and (2) a parenteral (intralesional) solution of interferon. Although interferon by itself has effectiveness in treating condylomata acuminata, lesser amounts of interferon are needed when it is used with podophyllin according to this invention. An active constituent of podophyllin, e.g., podophyllotoxin, can be used instead of podophyllin.

The topical solution of podophyllin in tincture of benzoin, preferred for use in this invention, contains about 25% by weight of podophyllin, though concentrations of from 15% to 30% also are suitable, and the parenteral interferon solution preferred for use in this invention contains a sufficient amount of interferon to provide about 1.5 Mu per lesion ($1.5 \times 10^6$ International Units) although amounts from $1 \times 10^4$ to $1 \times 10^7$ are suitable.

In addition, this invention provides a method of treating condylomata accuminata by topically administering to a patient in need of such treatment at the site of condylomata accuminata lesions (genital warts) a pharmaceutically acceptable topical composition of podophyllin or active constituent thereof in conjunction with intralesional administration of a pharmaceutically acceptable composition of human alpha-interferon. Recombinant-DNA human alpha-interferons as well as non-recombinant alpha- interferons are appropriate for use in this invention.

The active compounds can be adminstered in any effective sequence or essentially concurrently.

Administration of the podophyllin or active constituent thereof can be by any suitable topical means such as ointments, creams, solutions, lotions and the like; preferably the podophyllin (or active constituent) is administered as a solution.

The hIFNα-2b can be administered to the genital warts by any of the accepted modes of administration for interferons. These methods include parenteral, topical, depot and transdermal. Intralesional injection is preferred.

Injectable pharmaceutically acceptable and administrable alpha interferon is prepared by standard methods, e.g., by dissolving or dispersing the interferon in a pharmaceutically acceptable carrier, such as, for example, water, saline, aqueous dextrose, glycerol, ethanol, and the like to form a solution or suspension for injection. If desired, the injectable composition to be administered may also contain minor amounts of nontoxic auxiliary substances such as wetting or emulsifying agents, preservatives, pH buffering agents, and the like, for example, sodium acetate or sorbitan monolaurate. Actual methods of preparing such dosage forms are known, or will be apparent, to those skilled in this art; for example, see Remington's Pharmaceutical Sciences, Mack Publishing Company, Easton, Pennsylvania, 15th Edition (1975). The composition or formulation to be administered will, in any event, contain a quantity of interferon in an amount effective to achieve the desired effect in the subject being treated. A suitable human alpha-interferon injectable composition is available as Intron® A from Schering Corporation, Kenilworth, New Jersey. The commercial composition contains human interferon alfa-2b, glycine, di- and mono-basic sodium phosphate and human serum albumin.

The podophyllin is normally used as a tincture of benzoin in a pharmaceutical topical carrier. The amount of podophyllin is generally about 25% by weight. Additional additives and excipients which enhance the absorbability and substantivity of the podophyllin when applied topically can be incorporated into the topical podophyllin formulations. Those materials which are compatible with podophyllin are known to the ordinarily skilled artisan. A topical podophyllin composition suitable for use in this invention is Podofin RX available from Syosset Labs in Syosset, New York.

Based on the judgment of the attending clinician, the amount of the active components administered will, of course, be dependent on the subject being treated, the severity of the condylomata acuminata and the tolerance of the patient to the treatment regimen.

Administration in combination of topical podophyllin and intralesional recombinant alpha interferon is more efficacious than administration of podophyllin alone.


DETAILED DESCRIPTION


The following is a description of the clinical protocol utilized.


STUDY SYNOPSIS

The clinical study was a randomized, thirdparty blinded (i.e., "blind" evaluator), bicentric, parallel group design in a total of 108 patients. [A "blind" evaluator does not know which patient is receiving which treatment.]

All patients had three condylomata acuminata lesions treated and evaluated. The patients were randomized so that all three lesions (test sites) selected for treatment in a given individual received either an intralesional injection of interferon followed by topical application of podophyllin, or all three test sites received only a topical application of podophyllin. The test sites were treated once weekly for three weeks.

The amount of interferon administered was about $1.5 \times 10^6$ International Units and the amount of podophyllin was a 25% solution in tincture of benzoin sufficient to cover the lesion.

Patients with more than three lesions had those lesions (non-test sites) treated with podophyllin at the same time as their test sites were treated.

The size and symptomatology of the test sites were evaluated immediately prior to each treatment and day 1 of post-treatment weeks 1, 2, 4 and 11. A global evaluation reflecting overall change of non-test sites from pre-treatment was obtained at each evaluation after the first.

At each evaluation after the first, the global (overall) change of each test site relative to the condition at entry into the study was estimated and adverse reactions noted. Blood chemistry, hematology and urinalysis were also conducted to check for systemic side effects.

A third party evaluator, i.e. one who was unaware of the treatment assigned, made all efficacy and safety evaluations.


## STUDY DESIGN


This was a randomized, third party blinded (blind evaluator), bicentric parallel group study designed to determine if treating condyloma acuminatum (genital warts caused by the human papilloma virus) once weekly for three weeks with the combination of topical podophyllin (25% in tincture of benzoin) and recombinant-DNA human alpha interferon ($1.5 \times 10^6$ IU) intralesionally is more efficacious than podophyllin alone. 97 patients with anogenital warts completed the study. 49 of the patients were treated with the combination of hIFNα°-2b and podophyllin whereas 48 were treated with podophyllin alone.

In the study three lesions (venereal warts) with their smallest diameter about 4 mm and their largest diameter about 16 mm were selected as test sites. The test sites were outlined with a skin marking pen and designated A, B and C respectively. Each test site was evaluated, photographed and the location documented on a dermogram.

For a given patient, each of the test sites received either (1) an intralesional injection of 0.15 cc containing $1.5 \times 10^6$ International Units hIFNα-2b followed by topical application of 25% podophyllin in tincture of benzoin, or (2) only topical application of 25% podophyllin in tincture of benzoin. The interferon injection was made into the base and substance of the test site lesions using a 30 gauge needle. Although different concentrations of the interferon can be used, the dosage used in the study is preferred since it is efficacious.

The podophyllin was topically applied to the test sites and allowed to dry before the subject left the office. The subjects were instructed to wash off the podophyllin with mild soap and water four to six hours after each application unless severe local intolerance developed, when the podophyllin was washed off sooner. Although a 25% solution of podophyllin in tincture of benzion is preferred, weaker solutions can be used. However, the use of more concentrated solutions results in skin irritation.

During the three-week treatment period, the size and global response of each test site were evaluated immediately prior to each treatment. Photographs of the test sites were taken at these times to document changes in disease status or local intolerance to the treatments. Also, at each visit all symptoms and adverse reactions which occurred since the previous evaluations were recorded.

After treatment was completed on day 1 of week 3, the test sites were evaluated on day 1 of weeks 4, 5, 7 and 11; throughout weeks 4 to 11, no treatment was given. During the evaluation, the size of the lesions, side effects and global responses were observed and the test sites were photographed. A global evaluation reflecting overall change of non-test sites from their pretreatment status was also obtained.


## DRUG SUPPLIES


4

Recombinant-DNA human interferon alfa-2b was supplied by Schering Corp., Kenilworth, New Jersey in vials containing $1.0 \times 10^7$ International Units of recombinant-DNA human interferon alfa-2b in a lyophilized powder containing glycine, phosphate buffers (USP) and human serum albumin (HSA). One milliliter of sterile water for injection was added to each vial just prior to use. The powder was dissolved in the water. For each injection 0.15 cc of the solution (isotonic) was drawn into a syringe and injected. 0.15 cc of the solution provides $1.5 \times 10^6$ IU of the interferon.

Podophyllin was obtained as the topical composition Podofin RX from Syosset Laboratories, Syosset, New York.

## EFFICACY EVALUATIONS

Just prior to each subjects' first treatment, the size of each test site's largest perpendicular diameter and average height was measured in millimeters. The patient rated the symptoms, e.g. burning, itching and pain for each test site as follows: 1 = mild, 2 = moderate, 3 = severe. Color photographs of the test sites were taken at an appropriate magnification.

On day 1 of weeks 2 and 3, prior to the treatment that day, the size of each test site was again recorded along with a global evaluation of the change in the condition of each test site which occurred since the start of treatment, using the criteria in the six point scale shown in Table 1. The global evaluation of change for each test site was based on the criteria listed in Table 1 and reflected the "blind" evaluator's overall observations regarding flattening, change in volume, change in consistency or other physical characteristics of that lesion. Photographs were taken of the test sites at each evaluation.

Post-treatment evaluations were carried out 1, 2, 4 and 8 weeks after the last treatment, i.e. day 1 of study weeks 4, 5, 7 and 11. The evaluations consisted of determining size, evaluating global response and photographing the test sites. All global evaluations for each test site in the eighth week post-treatment evaluation (study week 11) were directly compared to the pictures depicting the initial, i.e. pretreatment, status of the lesion.

The following Table 1 is the scale for global evaluation of change in condylomata acuminata lesions:

TABLE 1

| Scale for Global Evaluation of Change in Condylomata | | |
| --- | --- | --- |
| Score | Descriptive Term | Definition |
| 1 | Cleared | 100% clearance of the lesion |
| 2 | Marked Improvement | >75% to <100% reduction in lesion mass |
| 3 | Moderate Improvement | >50% to <75% reduction in lesion mass |
| 4 | Slight Improvement | <50% reduction in lesion mass |
| 5 | No Change | No change from baseline evaluation |
| 6 | Exacerbation | Increase in lesion mass |

The results of the study are presented in the following Tables.

The data in Table 2 show that in each week the combination treatment resulted in more patients having all three test sites cleared of lesions than treatment with podophyllin alone.

TABLE 2

| Treatment | Week | Number of Lesions Cleared (Patients) | | | |
|---|---|---|---|---|---|
| | | 0 | 1 | 2 | 3 |
| Interferon Plus Podophyllin | 2 | 24 | 8 | 6 | 11 |
| | 3 | 12 | 7 | 7 | 23 |
| | 4 | 5 | 8 | 5 | 23 |
| | 5 | 5 | 5 | 6 | 25 |
| | 7 | 11 | 8 | 5 | 20 |
| | 11 | 19 | 6 | 7 | 12 |
| Podophyllin alone | 2 | 25 | 10 | 6 | 7 |
| | 3 | 20 | 9 | 8 | 11 |
| | 4 | 18 | 4 | 5 | 18 |
| | 5 | 19 | 5 | 6 | 14 |
| | 7 | 22 | 5 | 10 | 9 |
| | 11 | 28 | 9 | 3 | 5 |

In addition, the total number of patients With at least one lesion test site cleared at the eighth week post treatment (week 11) was 47% greater for concomitantly treated patients than for patients treated with podophyllin alone.

At week 11, 57% of the patients treated with the combination had at least one lesion test-site clear whereas only 38% of patients treated with podophyllin alone had at least one lesion cleared.

The data in Table 3 show that more patients had a greater percent reduction in the size of lesions when treated with interferon plus podophyllin than those treated with only podophyllin, particularly at weeks 5, 7 and 11. For example, at week 5, 39 of 41 patients showed a reduction of lesion size greater than 50% when undergoing combination treatment, whereas only 37 of 44 patients treated with podophyllin alone showed such a reduction.

Table 3

| Percent reduction of size of lesions at test sites from the pre-treatment measurements | | | | | | | |
|---|---|---|---|---|---|---|---|
| Treatment | Week | <0 | 0-25 | 26-50 | 51-75 | 76-99 | 100 |
| Interferon plus podophyllin | 2 | 0 | 2 | 2 | 14 | 20 | 11 |
| | 3 | 0 | 0 | 0 | 7 | 19 | 23 |
| | 4 | 0 | 0 | 1 | 1 | 16 | 23 |
| | 5 | 0 | 0 | 2 | 3 | 11 | 25 |
| | 7 | 2 | 0 | 4 | 8 | 10 | 20 |
| | 11 | 3 | 4 | 7 | 12 | 6 | 12 |
| Podophyllin alone | 2 | 0 | 6 | 6 | 9 | 20 | 7 |
| | 3 | 0 | 3 | 3 | 8 | 23 | 11 |
| | 4 | 0 | 0 | 5 | 5 | 17 | 18 |
| | 5 | 1 | 2 | 4 | 5 | 18 | 14 |
| | 7 | 4 | 8 | 3 | 8 | 14 | 9 |
| | 11 | 8 | 7 | 8 | 8 | 9 | 5 |

In addition, the mean percent reduction in lesion size compared to the pre-treatment lesion size was as follows:

Table 4

| Treatment | Week | Mean Size Reduction (%) |
|---|---|---|
| Interferon plus podophyllin | 2 | 79.4 |
| | 3 | 92.2 |
| | 4 | 94.2 |
| | 5 | 93.7 |
| | 7 | 80.0 |
| | 11 | 63.0 |
| Podophyllin alone | 2 | 71.6 |
| | 3 | 81.1 |
| | 4 | 85.7 |
| | 5 | 75.8 |
| | 7 | 56.3 |
| | 11 | 28.8 |

The data in Table 4 show that the reduction in lesion size peaked at weeks 4 and 5, after which some recurrence appeared. However in all weeks the combined treatment resulted in more lesion reduction.

Side effects occurred in more patients treated with interferon and podophyllin than with podophyllin alone; however, most of the side effects which were attributed to the interferon were alleviated by administering acetaminophen. The side effects had very little influence on the clinical program.

Clearly the results in the Tables above show that the combination treatment of this invention is more efficacious than treatment with podophyllin alone.

The invention further provides a kit comprising separate pharmaceutical compositions for use in the method of treating condyloma acuminata infections as defined above, said kit comprising (a) a first pharmaceutical composition comtaining as active ingredient human alpha interferon, and (b) a second pharmaceutical compostion containing as active ingredient podophyllin or an active constituent thereof.

**Claims**

1. A method of treating condylomata acuminata infections in patients in need of such treatment comprising administering in combination an effective amount of human alpha-interferon and an effective amount of podophyllin, or an active constituent thereof, to the lesions.

2. A method as claimed in claim 1 wherein the human alpha interferon is recombinant human DNA interferon alfa-2b.

3. A method as claimed in claim 1 wherein the human alpha interferon is recombinant human DNA interferon alfa-2a.

4. A method as claimed in claim 1 wherein the active constituent is podophyllotoxin.

5. A method as claimed in claim 1 wherein the treatment is sufficiently frequent and for a sufficient length of time to be efficacious.

6. A method as claimed in claim 1 wherein the treatment is once a week for three weeks.

7. A method as claimed in claim 2 wherein the treatment is once a week for three weeks.

8. A method as claimed in claim 3 wherein the treatment is once a week for three weeks.

9. A method as claimed in claim 1 wherein the human alpha-interferon is administered first.

10. A method as claimed in claim 1 wherein the podophyllin or active constituent thereof is administered first.

11. A method as claimed in claim 1 wherein the human alpha-interferon is administered by intralesional injection and the podophyllin is administered topically.

12. A method as claimed in claim 9 wherein the human alpha-interferon is recombinant human DNA interferon alfa-2b.

13. Use of podophyllin, or an active constituent thereof, in the manufacture of a pharmaceutical composition for treating condyloma acuminatum infections by the method defined in any one preceding claim.

14. Use of human alpha-interferon in the manufacture of a pharmaceutical composition for treating condyloma acuminatum infections by the method defined in any one of claims 1 to 12.

15. Use of human alpha-interferon for the preparation of a pharmaceutical composition for the intralesional treatment of condyloma acuminatum infections in conjunction with topical treatment with podophyllin or with an active constituent thereof.

16. Use of podophyllin or an active constituent thereof for the preparation of a pharmaceutical composition for the topical treatment of condyloma acuminatum infections in conjunction with intralesional treatment with human alpha-interferon.

17. A kit comprising separate pharmaceutical compositions for use in the method of treating condyloma acuminata infections as defined in any one of claims 1 to 11, said kit comprising (a) a first pharmaceutical composition comtaining as active ingredient human alpha interferon, and (b) a second pharmaceutical compostion containing as active ingredient podophyllin or an active constituent thereof.

European Patent Office

# PARTIAL EUROPEAN SEARCH REPORT

which under Rule 45 of the European Patent Convention shall be considered, for the purposes of subsequent proceedings, as the European search report

Application number

EP 89 30 6686

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | EP-A-0 119 852 (PHARMA-MEDICA AS)<br><br>* Whole document *<br><br>------ | 13-17 | A 61 K 45/02//<br>(A 61 K 45/02,<br>31:365) |

TECHNICAL FIELDS
SEARCHED (Int. Cl.4)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 13-17

Claims searched incompletely:

Claims not searched: 1-12

Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 06-10-1989 | BRINKMANN |